(19) Europäisches Patentamt
European Patent Office
Office européen des brevets

(11) **EP 1 075 827 B1**

(12) **EUROPÄISCHE PATENTSCHRIFT**

(45) Veröffentlichungstag und Bekanntmachung des Hinweises auf die Patenterteilung:
**15.11.2006 Patentblatt 2006/46**

(51) Int Cl.:
***A61F 13/00*** *(2006.01)*

(21) Anmeldenummer: **00115955.7**

(22) Anmeldetag: **26.07.2000**

(54) **Selbstklebender Formkörper**

Self-adhesive shaped body

Coquille moulée autocollante

(84) Benannte Vertragsstaaten:
**AT BE CH CY DE DK ES FI FR GB GR IE IT LI LU MC NL PT SE**

(30) Priorität: **12.08.1999 DE 19938322**

(43) Veröffentlichungstag der Anmeldung:
**14.02.2001 Patentblatt 2001/07**

(73) Patentinhaber: **Beiersdorf AG**
**20253 Hamburg (DE)**

(72) Erfinder:
• **Himmelsbach, Peter**
**21614 Buxtehude (DE)**

• **Herzberg, Thorsten**
**22419 Hamburg (DE)**

(56) Entgegenhaltungen:
**US-A- 3 934 583**

• **PATENT ABSTRACTS OF JAPAN vol. 018, no. 233 (C-1195), 28. April 1994 (1994-04-28) & JP 06 022999 A (SEKISUI CHEM CO LTD;OTHERS: 02), 1. Februar 1994 (1994-02-01)**

• **PATENT ABSTRACTS OF JAPAN vol. 017, no. 131 (C-1036), 18. März 1993 (1993-03-18) & JP 04 305522 A (SEKISUI CHEM CO LTD), 28. Oktober 1992 (1992-10-28)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft einen selbstklebenden Formkörper, auch als Pelotte bezeichnet, mit hoher Rückstellkraft für orthopädische Zwecke, insbesondere zur Prophylaxe, postoperativen Behandlung und Therapie, speziell zur schnelleren Wiederherstellung der Funktion von verletzten Gelenken, wie Knie-, Sprung-, Schulter- Ellenbogen- und/oder Handgelenken.

Das Bandagieren von Gelenken, zum Beispiel nach Verletzung oder bei degenerativen Erkrankungen, ist eine bekannte orthopädische Behandlungsmethode. Hierbei reicht das Spektrum vom einfachen Wickeln mit mehr oder weniger elastischen Binden, über Fertigbandagen in den verschiedensten Ausführungsformen bis zu aufwendig konstruierten Schienen und Braces.

Ferner ist die funktionelle Verbandtechnik, das sogenannte Taping, eine Behandlungsmethode zur Prophylaxe und Therapie von Verletzungen, Krankheiten und Veränderungen am Bewegungsapparat. Taping hat zum Ziel, die Kapsel-Band-Strukturen gezielt nachzubilden und dadurch eine selektive Unterstützung und Stabilisierung zu erreichen.

Der eigentliche Tapeverband wird dabei streifenweise aus vorzugsweise unelastischen selbstklebenden Bändern, sogenannten Zügeln, oder in Verbindung mit kurzzugelastischen selbstklebenden Bändern angelegt. Er schützt, stützt und entlastet gefährdete, geschädigte oder gestörte Anteile einer Funktionseinheit. Er erlaubt die selektive Belastung im schmerzfreien Bewegungsraum, verhindert aber extreme oder schmerzhafte Bewegungen.

Das Anlegen derartiger Verbände erfordert jedoch fachmännisches Können und Erfahrung und kann deshalb in aller Regel nicht von Laien ohne Taping-Erfahrung ausgeführt werden.

Bekannt ist eine Vielzahl von Bandagen, die sich der Wiederherstellung der Funktion von verletzten Gelenken widmen.

EP 0 027 172 A1 beschreibt eine Bandage in Schlauchform zur Abstützung beziehungsweise Kompression von Knie-, Sprung-, Ellenbogen- und/oder Handgelenken. Eine klebende Beschichtung der Bandage ist nicht beschrieben.

Eine Epicondylitis-Bandage ist durch die EP 0 250 409 A1 bekannt. Eine derartige Epicondylitis-Bandage besteht im wesentlichen aus einem Schlauchabschnitt aus elastischem Material, wobei die Zugspannung in Umfangsrichtung über einen im wesentlichen in Umfangsrichtung verlaufenden Spannriemen mit Verschluß veränderbar ist. Die Bandage ist nicht selbstklebend ausgerüstet.

DE 41 01 965 A1 offenbart eine Gelenkbandage aus elastischem Bandagenmaterial mit einem weichen und einem harten Anteil und mindestens einem Friktionskern. Selbstklebende Eigenschaften sind nicht beschrieben.
Die Gelenkbandage ermöglicht eine gezielte Querfriktionsmassage zur schnelleren Wiederherstellung der Gelenkfunktionen von verletzten Gelenken wie Knie-, Sprung-, Ellenbogen-, Schulter- und Handgelenken und weist eine im angelegten Zustand das Gelenk beaufschlagende Einlage auf, die als Druckpelotte ausgebildet ist, die eine durch die Knochenvorsprünge und Sehnenansätze des Gelenkes bestimmende Form aufweist und als Formkörper aus einem weichen oder weich-elastischen Material ausgebildet ist, in dem mindestens ein Friktionskern aus einem harten oder inkompressiblen Material angeordnet und in dem Material des Formkörpers in seiner Lage fixiert ist.

DE 42 37 389 A1 beschreibt eine Bandage für das Kniegelenk, welche entsprechende anatomische Formen aufweist. Selbstklebende Eigenschaften sind nicht beschrieben.
Die Bandage für Überlastungserscheinungen, femoropatellare Schmerzsyndrome und das Patellaspitzensyndrom aus einem elastischen Bandagenstoff in Schlauchform mit einem rundherum laufenden Einsatz aus einem Wellengestrick weist in dem vorderen Bandagenteil eine im Bereich über der Kniescheibe bei angelegter Bandage liegende, nach oben offene und die Quadrizeps-Sehne freilassende Pelotte und in dem rückwärtigen Bandagenteil eine oder zwei im Abstand voneinander angeordnete, den Ansatz der ischiokruralen Muskulatur beaufschlagende Pelotten auf.

Patent Abstracts of Japan Vol. 018, No. 233 vom 28.04-1994 und JP 06022999 vom 01.02.1994 offenbaren ein Pflaster bestehend aus einem geschäumten Material, dass aus Polyethylen, Ethylen-vinylacetat Copolymer, Polyurethan, Polypropylen und Polyvinylchloride bestehen kann. Auf der oberfläche dieses Materials ist ein zusätzliche Adhäsionsschicht aufgebracht um eine Verklebung zu ermöglichen. Das Material hat eine Dicke von 0,2 bis 7 mm. Das hierin offenbarte Pflaster ist weder selbstklebend noch ein der Anatomie angepasster Körper.

Aufgabe der Erfindung ist es, einen anatomisch ausgebildeten Formkörper für orthopädische Zwecke bereitzustellen, welcher aufgrund der selbstklebenden Eigenschaften auf der Haut oder auf einem Unterzugmaterial haftet, wodurch sich aufgrund des hohen Rückstellvermögens des Formkörpers bei richtiger Anwendung eine Massagewirkung einstellt, welche zu einer schnelleren Wiederherstellung der Funktion von verletzten Gelenken, wie Knie-, Sprung-, Schulter- Ellenbogen- und/oder Handgelenken führt. Weiter soll die propriorezeptive Wirkung durch den Formkörper verbessert werden.

Gelöst wird die Aufgabe durch einen selbstklebenden, der Anatomie angepaßten Formkörper mit propriorezeptiver Wirkung für medizinische Zwecke wie er im Hauptanspruch beschrieben ist. Gegenstand der Unteransprüche stellen vorteilhafte Ausführungsformen der Erfindung dar.

Demgemäß betrifft die Erfindung einen selbstklebenden, der Anatomie angepasster Formkörper für medizinische Zwecke mit einer Dicke von mehr als 0,5 mm bestehend aus einem elastischen Polymer gewählt aus der Gruppe Polyurethan, Polyester, Polyether, Polyepoxid, Polyolefin und/oderauf Basis eines natürlichen oder synthetischen Kautschuk, wobei dem Polymer 5 - 80 Gew.% Klebrigmacher, wie Öle, Wachse, Harze und/oder deren Mischungen, weniger als 60 Gew.% Weichmacher, weniger als 15 Gew.% Additive und weniger als 5 Gew.% Stabilisatoren zugesetzt sind. Der Formkörper weist dadurch

- eine Klebkraft von 1 N/cm bis 25 N/cm,
- eine Dehnfähigkeit von mehr als 10 %,
- eine Dicke von mehr als 0,5 mm und
- ein Rückstellvermögen von mehr als 50% bei einer Zugbeanspruchung von 5 N/cm$^2$. auf.

Der erfindungsgemäße Formkörper basiert auf einer Zusammensetzung, welche durch entsprechende Verfahrensschritte oder durch Rezeptierung mit Klebrigmachem selbstklebrig ausgebildet ist.

Je nach Einsatzgebiet lassen sich die technischen Eigenschaften des Formkörpers einstellen. Fallweise werden stark klebende Systeme oder aber auch weniger haftklebrige Systeme für den Formkörper benötigt. Hierzu können dem jeweiligen System entsprechende Zusatzstoffe wie Klebharze, Weichmacher, Stabilisatoren und andere Hilfsstoffe zugesetzt werden.

Vorteilhafte Ausführungen zeigen eine Klebkraft von 1,5 bis 22 N/cm, besonders bevorzugt von 1,5 bis 18 N/cm. In einer speziellen Ausführung für das Knie beträgt die Klebkraft 14,5 N/cm. In einer weiteren speziellen Ausführung für den Ellenbogen beträgt die Klebkraft 16,0 N/cm.

In einer besonders bevorzugten Ausführung ist die zur Herstellung des Formkörpers eingesetzte Masse eine thermoplastische Schmelzmasse.

Der Erweichungspunkt der thermoplastischen Schmelzmasse sollte höher als 50 °C liegen, da die Applikationstemperatur bei der Herstellung in der Regel mindestens 70 °C beträgt, bevorzugt zwischen 90 °C und 190 °C. Gegebenenfalls kann eine Nachvemetzung durch UV- oder Elektronenstrahlen-Bestrahlung angebracht sein. Dieses hängt vom gewählten Aufbau des Stammpolymers oder dessen Zusatzstoffen ab.

Insbesondere die Abmischung von Blockcopolymeren auf Basis von SEPS und SEBS zur Herstellung eines erfindungsgemäßen Formkörpers zeichnet sich durch ihre vielfältigen Variationsmöglichkeiten aus.

Für besonders starkhaftende Systeme basiert die kohäsive Haftmasse bevorzugt auf Di-Block- (A-B-), Tri-Blockcopolymeren (A-B-A-Blockcopolymere) und/oder deren Mischungen, wobei ein Anteil an Di-Blockcopolymeren von kleiner 80 Gew.-% bevorzugt wird. Die harte Phase A ist vornehmlich Polystyrol oder dessen Derivate, und die weiche Phase B enthält Ethylen und Propylen beziehungsweise Butylen oder deren Mischungen.

Die Kette der Phase B kann auch artfremde Teile enthalten, wie zum Beispiel Isopren, Butadien oder ähnliche Stoffe. Polystyrolblöcke können aber auch in der weichen Phase B enthalten sein, und zwar bis zu 20 Gew.-%. Der gesamte Styrolanteil sollte aber stets niedriger als 65 Gew.-%, bevorzugt weniger als 40 Gew.-%, besonders bevorzugt 3 bis 35 Gew.-%, liegen. Bevorzugt werden Styrolanteile zwischen 3 Gew.-% und 35 Gew.-%, da ein niedrigerer Styrolanteil die Klebemasse anschmiegsamer macht.

In einer vorteilhaften Ausführung weist die Haftmasse die nachfolgend angegebene Zusammensetzung auf:

| | |
|---|---|
| 5 Gew.-% bis 90 Gew.-% | Blockcopolymere, |
| 5 Gew.-% bis 80 Gew.-% | Klebrigmacher wie Öle, Wachse, Harze und/oder deren Mischungen, bevorzugt Mischungen aus Harzen und Ölen, |
| weniger als 60 Gew.-% | Weichmacher, |
| weniger als 15 Gew.-% | Additive, |
| weniger als 5 Gew.-% | Stabilisatoren, |

Die als Klebrigmacher dienenden aliphatischen oder aromatischen Öle, Wachse und Harze sind bevorzugt Kohlenwasserstofföle, -wachse und -harze, wobei sich die Öle, wie Paraffinkohlenwasserstofföle, oder die Wachse, wie Paraffinkohlenwasserstoffwachse, durch ihre Konsistenz günstig auf die Haftwirkung auswirken. Als Weichmacher finden mittel- oder langkettige Fettsäuren und/oder deren Ester Verwendung. Diese Zusätze dienen dabei der Einstellung der Klebeeigenschaften und der Stabilität. Gegebenenfalls kommen weitere Stabilisatoren und andere Hilfsstoffe zum Einsatz.

Ein Füllen der kohäsiven Haftmasse mit mineralischen Füllstoffen, Fasern, Mikrohohl- oder -vollkugeln ist möglich, so daß sich beispielsweise ein solcher Körper aus einem harten Kern und einer weichen Peripherie ergibt. Dieses kann in der Anwendung eine Tiefenmassage hervorrufen.

Vorteilhaft sind für spezielle Anwendungen Kemmaterialien mit einer Shore-Härte (A) von weniger als 90, bevorzugt 80 bis 10, besonders bevorzugt 70 bis 20. Der Römpp gibt zur Shore-Härte folgende Erläuterungen (Römpp Lexikon Chemie - Version 1.5, Stuttgart/New York: Georg Thieme Verlag 1998) an:

Nach DIN 53505 (1987-06) entspricht die Shore-Härte bei der Prüfung von Elastomeren, Gummi und Kautschuk dem Widerstand gegen das Eindringen eines Kegelstumpfes (A oder C) beziehungsweise eines abgerundeten Kegels (D), der durch das Zusammendrücken einer Feder mit einer festgelegten Federcharakteristik gemessen und in dimensionslosen Shore-A(C, D)-Härteeinheiten ausgedrückt wird. Bei der Prüfung von Stahl wird die Shore-Rückprallhärte in dem sogenannten Skleroskop gemessen, bei dem die Rückprallhöhe eines Fallhammers, der in einem senkrechten Rohr auf die Prüffläche fällt, bestimmt wird.

In weiteren Ausführungen ist dieses Kernmaterial nicht klebend ausgelegt.

Weiter können für spezielle Anwendung auch Naturkautschuk oder andere Synthesekautschukarten, wie Butylkautschuk, Chloropren, Silikonkautschuk verwendet werden.

Insbesondere für beanspruchte Gelenkbereiche werden hohe Anforderungen bezüglich der Klebeeigenschaften gestellt. Für eine ideale Anwendung sollte der Formkörper eine hohe Anfaßklebrigkeit aufweisen. Weiterhin ist, damit es zu keinem Verrutschen kommt, eine hohe Scherfestigkeit der zur Herstellung des Formkörpers gewählten Haftklebemasse notwendig.

Durch die gezielte Absenkung der Glasübergangstemperatur der Haftklebemasse infolge der Auswahl der Klebrigmacher, der Weichmacher sowie der Polymermolekülgröße und der Molekularverteilung der Einsatzkomponenten wird die notwendige funktionsgerechte Verklebung auf der Haut auch an kritischen Stellen des menschlichen Bewegungsapparates gewährleistet und oder auf einem Unterzugmaterial erreicht.

Die Produkteigenschaften wie Anfaßklebrigkeit und Scherstabilität lassen sich mit Hilfe einer dynamisch-mechanischen Frequenzmessung gut quantifizieren. Hierbei wird ein schubspannungsgesteuertes Rheometer verwendet.

Die Ergebnisse dieser Meßmethode geben Auskunft über die physikalischen Eigenschaften eines Stoffes durch die Berücksichtigung des viskoelastischen Anteils. Hierbei wird bei einer vorgegebenen Temperatur die kohäsive Haftmasse zwischen zwei planparallelen Platten mit variablen Frequenzen und geringer Verformung (linear viskoelastischer Bereich) in Schwingungen versetzt. Über eine Aufnahmesteuerung wird computerunterstützt der Quotient ($Q = \tan \delta$) zwischen dem Verlustmodul (G'' viskoser Anteil) und dem Speichermodul (G' elastischer Anteil) ermittelt.

$$Q = \tan \delta = G''/G'$$

Für das subjektive Empfinden der Anfaßklebrigkeit (Tack) wird eine hohe Frequenz gewählt sowie für die Scherfestigkeit eine niedrige Frequenz.

Ein niedriger Zahlenwert bedeutet eine geringe Anfaßklebrigkeit und eine gute Scherstabilität.

| Bezeichnung | Scherfestigkeit niedrige Frequenz/RT | Anfaßklebrigkeit hohe Frequenz/RT |
|---|---|---|
| Haftklebemasse A | $\tan \delta = 0{,}12 \pm 0{,}03$ | $\tan \delta = 1{,}0 \pm 0{,}1$ |
| Haftklebemasse B | $\tan \delta = 0{,}37 \pm 0{,}03$ | $\tan \delta = 1{,}7 \pm 0{,}1$ |

Bevorzugt werden erfindungsgemäß Haftklebemassen zur Bildung des Formkörpers, bei denen das Verhältnis des viskosen Anteils zum elastischen Anteil bei einer Frequenz von 100 rad/s bei 25 °C größer 0,4 ist, oder Haftmassen, bei denen das Verhältnis des viskosen Anteils zum elastischen Anteil bei einer Frequenz von 0,1 rad/s bei 25 °C kleiner 0,5 ist, bevorzugt zwischen 0,35 und 0,02, ganz besonders bevorzugt zwischen 0,3 und 0,05. Die Klebemassen sind weiter vorzugsweise so eingestellt, daß sie bei einer Frequenz von 0,1 rad/s eine dynamisch-komplexe Glasübergangstemperatur von weniger als 25 °C, bevorzugt von weniger als 10 °C, ganz besonders bevorzugt von 5 °C bis -150 °C, aufweisen.

Aufgrund des erfindungsgemäß ideal gewählten Verhältnisses zeigt sich für die Pelotten ein günstiges Rückstellverhalten. Bei einer bevorzugten Ausführung ergibt sich bei einer Zugbeanspruchung von 5 N/cm, bezogen auf die Artikelbreite, ein Rückstellverhalten von mehr als 50 %, bevorzugt von 55 % bis 98 %, besonders vorteilhaft 60 % bis 90 %. Eine Ermüdung des Materials, wie sie zum Beispiel bei einer Sportbelastung auftritt, wird so reduziert.

Die hohe Scherfestigkeit der Haftmasse wird durch den kohäsiven Charakter des Polymeren, insbesondere Blockcopolymeren, erreicht.

Eine weiterreichende Verbesserung gegenüber dem bekannten Stand der Technik kann durch die Bereitstellung von dehnfähigen Ausführungen der Pelotten erreicht werden. Hierbei zeigt sich für eine dauerhafte Anwendung eine Verbesserung der propriorezeptiven Wirkung, denn zu geringdehnende Pelotten verkleben nicht dauerhaft und wirken somit nicht oder deutlich geringer propriorezeptiv.

Die Dehnfähigkeit hängt von der entsprechenden Verwendung des Produktes ab. Generell sind Dehnungen von mehr als 10 % notwendig. Für eine spezielle Ausführung am Knie sollte die Mindestdehnbarkeit 70 %, für eine andere spezielle Ausführung für die Verwendung am Ellenbogen 50 % betragen.

In einer besonderen Ausführung ist der Formkörper durch Dehnung entklebbar, auch hierfür ist eine gute Dehnfähigkeit des Pelottenmaterials notwendig.

Eine weitere besondere Ausführung der Formkörper ist mit freisetzbaren lokal oder systemisch wirkenden Stoffen dotiert.

In einem speziellen Fall ist der durch Dehnung entklebbare Formkörper mit einem an die Haut freisetzbaren Wirkstoff versetzt worden.

Die Herstellung der Formkörper kann aus der Schmelze der o.a. Masse erfolgen. Hierbei können verschiedene Herstellungsverfahren in Betracht kommen, wodurch sich je nach Verwendungszweck Vorteile ergeben können. Gängige Herstellungsarten sind das Gießen, Drucken, Spritzen, Verdüsen, Extrudieren, Kalandem sowie Stanzen, Prägen, Rekken, Schneiden oder deren Kombination.

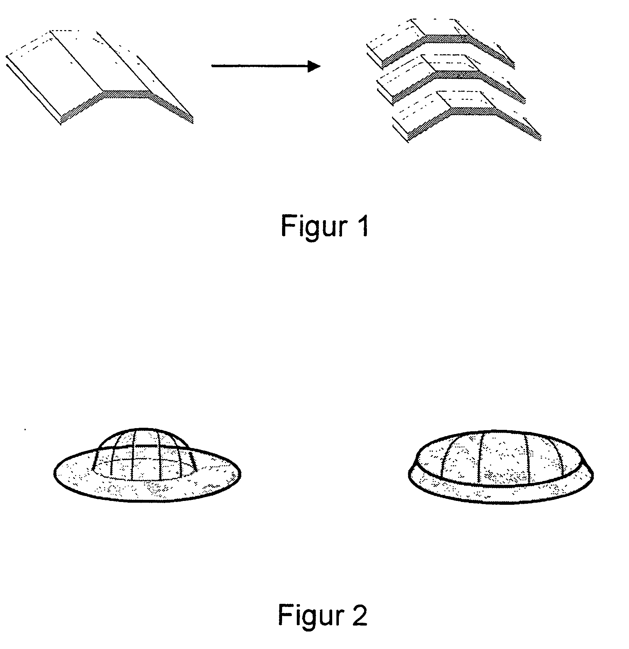

Für flache Formkörper können Profile extrudiert werden, wie sie beispielsweise die Figur 1 zeigt. Anschließend kann ein Schneiden der extrudierten Profile in Scheiben erfolgen.

Fallweise ist hier vor dem Schneiden ein Abkühlen vorteilhaft.

Eine weitere Herstellungsart ist das Formgießen. Hier wird in eine abhäsiv ausgerüstete Form die geschmolzene Masse eingefüllt. Vorteilhaft kann dieses unter Druck geschehen. Nach dem Verfestigen der Schmelze wird das Formteil aus der Form entfernt und eine neue Belastung kann beginnen. Mögliche Formen derartig erzeugter Formkörper sind in der Figur 2 dargestellt.

Für spezielle anatomische Spezialformen kann das Formteil auch aus einer räumlich begrenzten Platte oder einem endlosen Band gestanzt oder geschnitten werden.

Ein kontinuierliches Verfahren ist das Rasterdruckverfahren. In diesem wird über ein Rakel die Schmelze in Vertiefungen eingefüllt und an einer anderen Stelle aus der Vertiefung herausgenommen. Überlicherweise werden in diesem Verfahren Hilfsträger eingesetzt, auf denen die Formkörper vorübergehend aufgebracht werden. Hierdurch entstehen genoppte Oberflächen, welche einen besseren Massageeffekt aufweisen.

Bei speziellen Anwendungen des Formkörpers kann das vorherige Schäumen der Klebemassen desselben vorteilhaft sein.

Die Klebemassen werden dabei bevorzugt mit inerten Gasen wie Stickstoff, Kohlendioxid, Edelgasen, Kohlenwasserstoffen oder Luft oder deren Gemischen geschäumt. In manchen Fällen hat sich ein Aufschäumen zusätzlich durch thermische Zersetzung gasentwickelnder Substanzen wie Azo-, Carbonat- und Hydrazid-Verbindungen als geeignet erwiesen.

Der Schäumungsgrad, d.h. der Gasanteil, sollte mindestens etwa 5 Vol.-% betragen und kann bis zu etwa 85 Vol.-% reichen. In der Praxis haben sich Werte von 10 Vol.-% bis 75 Vol.-%, bevorzugt 50 Vol.-%, gut bewährt. Wird bei relativ hohen Temperaturen von ungefähr 100 °C und vergleichsweise hohem Innendruck gearbeitet, entstehen sehr offenporige Klebstoffschaumschichten, die besonders gut luft- und wasserdampfdurchlässig sind. Weiter können die wenigstens teilweise offenporigen Formkörper Feuchtigkeit aufnehmen.

Die vorteilhafte Eigenschaft ist die gute Anschmiegsamkeit auch an unebenen Flächen durch die Elastizität und Plastizität des geschäumten Formkörpers.

Dieser Vorteil kommt besonders bei Knochenvorsprüngen (zum Beispiel am Sprunggelenk) zum Tragen.

Ein besonders geeignetes Verfahren zur Herstellung der erfindungsgemäß geschäumten Vorrichtung arbeitet nach dem Schaum-Mix-System. Hierbei wird die thermoplastische Klebemasse unter hohem Druck bei einer Temperatur über dem Erweichungspunkt (ungefähr 120 °C) mit den vorgesehenen Gasen wie zum Beispiel Stickstoff, Luft oder Kohlendioxid in unterschiedlichen Volumenanteilen (etwa 10 Vol.-% bis 80 Vol.-%) in einem Stator/Rotorsystem umgesetzt.

Während der Gasvordruck größer 100 bar ist, betragen die Mischdrucke Gas/Thermoplast im System 40 bis 100 bar, bevorzugt 40 bis 70 bar. Der so hergestellte Haftklebeschaum kann anschließend über eine Leitung in das Auftragswerk gelangen. Bei dem Auftragswerk finden handelsübliche Düsen, Extruder- oder Kammersysteme Verwendung.

Durch die Schäumung des Formkörpers und die dadurch entstandenen offenen Poren in der Masse sind bei Verwendung eines an sich porösen Trägers die mit der Vorrichtung beschichteten Produkte gut wasserdampf- und luftdurchlässig. Die benötigte Haftklebermenge wird erheblich reduziert ohne Beeinträchtigung und Wirkungsweise der Eigenschaften.

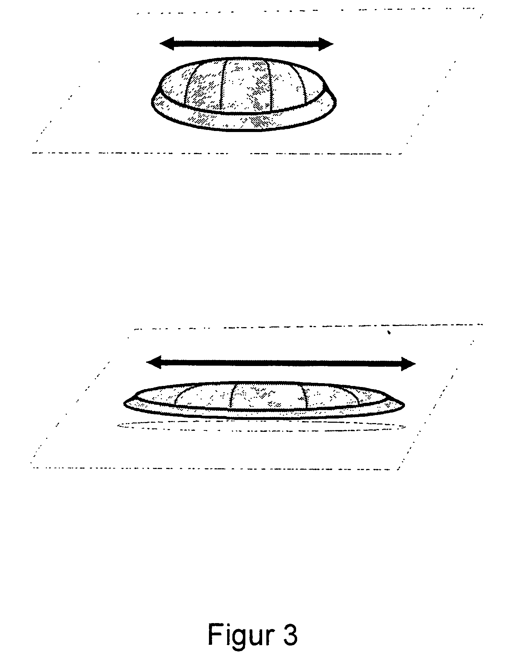

Die Figur 3 zeigt, daß der Formkörper in einer bevorzugten Ausführungsvariante durch streckendes Ziehen insbesondere in Richtung der Verklebungsebene wieder vom Untergrund - sprich insbesondere der Haut - lösbar ist.

**Patentansprüche**

1. Selbstklebender, der Anatomie angepasster Formkörper für medizinische Zwecke mit einer Dicke von mehr als 0,5 mm bestehend aus einem elastischen Polymer gewählt aus der Gruppe Polyurethan, Polyester, Polyether, Polyepoxid, Polyolefin und/oder auf Basis eines natürlichen oder synthetischen Kautschuk, **dadurch gekennzeichnet, dass** dem Polymer 5 - 80 Gew.% Klebrigmacher, wie Öle, Wachse, Harze und/oder deren Mischungen, weniger als 60 Gew.% Weichmacher, weniger als 15 Gew.% Additive und weniger als 5 Gew.% Stabilisatoren zugesetzt sind.

**2.** Selbstklebender Formkörper nach Anspruch 1, **dadurch gekennzeichnet, dass** der Formkörper aus einem styrolhaltigen A/B oder A/B/A Blockcopolymer besteht.

**3.** Formkörper nach einem der vorangegangenen Ansprüche, **dadurch gekennzeichnet, dass** der Formkörper geschäumt ist.

**4.** Formkörper nach Anspruch 3, **dadurch gekennzeichnet, dass** der Formkörper einen Gasanteil von mindestens 5 Vol.-% aufweist.

**5.** Formkörper nach Anspruch 4, **dadurch gekennzeichnet, dass** der Formkörper einen Gasanteil von 10 Vol.-% bis 85 Vol.-% aufweist.

**6.** Formkörper nach einem der vorangegangenen Ansprüche, **dadurch gekennzeichnet, dass** der Formkörper wenigstens eine genoppte Fläche hat

**7.** Formkörper nach einem der vorangegangenen Ansprüche, **dadurch gekennzeichnet, dass** der Formkörper eine freisetzbare lokal oder systemisch wirkende Substanz beinhaltet.

## Claims

**1.** Self-adhesive shaped body which is adapted to the anatomy and is intended for medical purposes, having a thickness of more than 0.5 mm comprising an elastic polymer selected from the group consisting of polyurethane, polyester, polyether, polyepoxide and polyolefin, and/or based on a natural or synthetic rubber, **characterized in that** there have been added to the polymer 5%-80% by weight of tackifiers such as oils, waxes, resins and/or mixtures thereof, less than 60% by weight of plasticizers, less than 15% by weight of additives and less than 5% by weight of stabilizers.

**2.** Self-adhesive shaped body according to Claim 1, **characterized in that** the shaped body comprises a styrene-containing A/B or A/B/A block copolymer.

**3.** Shaped body according to one of the preceding claims, **characterized in that** the shaped body is foamed.

**4.** Shaped body according to Claim 3, **characterized in that** the shaped body has a gas content of at least 5% by volume.

**5.** Shaped body according to Claim 4, **characterized in that** the shaped body has a gas content from 10% by volume to 85% by volume.

**6.** Shaped body according to one of the preceding claims, **characterized in that** the shaped body has at least one stippled area.

**7.** Shaped body according to one of the preceding claims, **characterized in that** the shaped body comprises a releasable substance having a local or systemic action.

## Revendications

**1.** Corps façonné autocollant, adapté à l'anatomie, pour un usage médical, présentant une épaisseur supérieure à 0,5 mm et constitué d'un polymère élastique choisi parmi le groupe constitué du polyuréthanne, du polyester, du polyéther, du polyépoxyde, de la polyoléfine et/ou à base d'un caoutchouc naturel ou synthétique, **caractérisé en ce qu'**au polymère sont ajoutés 5-80 % en poids d'agent collant, comme des huiles, des cires, des résines et/ou leurs mélanges, moins de 60 % en poids d'agents plastifiants, moins de 15 % en poids d'additifs et moins de 5 % en poids de stabilisants.

**2.** Corps façonné autocollant suivant la revendication 1, **caractérisé en ce que** le corps façonné est constitué d'un copolymère bloc de A/B/A ou A/B contenant du styrène.

**3.** Corps façonné suivant l'une des revendications précédentes, **caractérisé en ce que** le corps façonné est transformé en mousse.

**4.** Corps façonné suivant la revendication 3, **caractérisé en ce que** le corps façonné présente une fraction gazeuse d'au moins 5 % en volume.

**5.** Corps façonné suivant la revendication 4,
**caractérisé en ce que** le corps façonné présente une fraction gazeuse de 10 % en volume à 85 % en volume.

**6.** Corps façonné suivant l'une des revendications précédentes, **caractérisé en ce que** le corps façonné présente au moins une face nopée.

**7.** Corps façonné suivant l'une des revendications précédentes, **caractérisé en ce que** le corps façonné contient une substance libérable à action locale ou systémique.

## Figur 1

## Figur 2

Figur 3